# EUROPEAN PATENT APPLICATION

(11) **EP 4 011 310 A1**
(43) Date of publication of application: **15.06.2022**
(21) Application number: 20849484.9
(22) Date of filing: 31.07.2020
(51) Int. Cl.: A61B 18/14, A61B 18/12

(54) **PROBE APPLICATION MODE SWITCHING SYSTEM AND METHOD, AND TUMOR THERAPY INSTRUMENT**

(30) Priority: 06.08.2019 CN 201910720763
(71) Applicant: Shenzhen Neumann Technology Co., Ltd., Shenzhen, Guangdong 518000 (CN)
(72) Inventor: LAI, Shen, Shenzhen, Guangdong 518000 (CN)
(74) Representative: Roberts, Peter David
(86) International application number: PCT/CN2020/106409
(87) International publication number: WO 2021/023127

(57) **Abstract**

The present application relates to a probe application mode switching system and method, and a tumor therapy instrument. The probe application mode switching system comprises an instruction acquisition module, and a control module connected to the instruction acquisition module, wherein the instruction acquisition module receives a probe application mode instruction, and transmits the probe application mode instruction to the control module; and the control module acquires a probe application mode to be switched to corresponding to the probe application mode instruction, and switches the current probe application mode to the probe application mode to be switched to, thereby achieving the switching of a probe application mode. In the present application, mode switching can be automatically performed according to a probe application mode instruction, thereby diversifying probe application manners and improving the convenience of the product.

## Description

### TECHNICAL FIELD

The present application relates to the technical field of medical devices, and particularly to a probe distribution mode switching system and method, and a tumor therapy instrument.

### BACKGROUND

With the development of science and technology, the application of tumor therapy instruments has received widespread attention. There are many types of tumor therapy instruments. As for the tumor therapy instruments based on the electric pulse technology, the tumor ablation process can be simulated through a simulation system, which usually requires the manual operation of the probe to ablate the tumor. Accordingly, the simulated probe distribution mode is single, and the operation has low convenience.

In the implementation process, the inventors found that the conventional technology has at least the following problems: the conventional simulation system simulates the tumor ablation by manually operating the probe, accordingly the probe distribution mode is single.

### SUMMARY

In view of this, as for the conventional simulation system which requires manual operation of the probe to perform the tumor ablation, the probe distribution manner is single. Thus, it is necessary to provide a probe distribution mode switching system and method, and a tumor therapy instrument.

In order to achieve the foregoing purpose, in an embodiment of the present invention, a probe distribution mode switching system is provided, including an instruction acquisition module and a control module connected to the instruction acquisition module; wherein
the instruction acquisition module is configured to receive a probe distribution mode instruction, and transmit the probe distribution mode instruction to the control module;
the control module is configured to acquire a to-be-switched probe distribution mode corresponding to the probe distribution mode instruction, and switch a current probe distribution mode to the to-be-switched probe distribution mode.

In an embodiment, the control module is configured to compare a priority of the to-be-switched probe distribution mode to a priority of the current probe distribution mode, and switch the current probe distribution mode to the to-be-switched probe distribution mode when the priority of the to-be-switched probe distribution mode is greater than the priority of the current probe distribution mode.

In an embodiment, the control module is configured to perform the current probe distribution mode when the priority of to-be-switched probe distribution mode is less than the priority of the current probe distribution mode.

In an embodiment, the to-be-switched probe distribution mode is an automatic probe distribution mode or a manual probe distribution mode.

In an embodiment, the control module is configured to parse the probe distribution mode instruction, and perform switching of a corresponding mode according to a parsing result.

In an embodiment, the control module is configured to, when acquiring an initialization instruction, start the automatic probe distribution mode.

In an embodiment, the system further includes a display module,
the display module is configured to display an automatic probe distribution interface corresponding to the automatic probe distribution mode, and display a manual probe distribution interface corresponding to the manual probe distribution mode.

In another aspect, a probe distribution mode switching method is provided, including:
receiving a probe distribution mode instruction;
acquiring a to-be-switched probe distribution mode corresponding to the probe distribution mode instruction, and switching a current probe distribution mode to the to-be-switched probe distribution mode.

In another aspect, a tumor therapy instrument is provided, including the probe distribution mode switching system according to any one of the above embodiments.

In an embodiment, the instrument further includes an input device configured to input the probe distribution mode instruction.

One of the above technical solutions has the following advantages and beneficial effects.

The probe distribution mode instruction is received by the instruction acquisition module; the control module acquires the to-be-switched probe distribution mode corresponding to the probe distribution mode instruction according to the probe distribution mode instruction; and then the control module can switch the current probe distribution mode to the to-be-switched probe distribution mode to implement the switching of the probe distribution mode. In the present application, the mode can be automatically switched according to the probe distribution mode instruction, thereby diversifying the probe distribution manners and improving the convenience of the product.

### BRIEF DESCRIPTION OF THE DRAWINGS

FIG. 1 is a schematic structure diagram I of a probe distribution mode switching system according to an embodiment.
FIG. 2 is a schematic structure diagram II of a probe distribution mode switching system according to an embodiment.
FIG. 3 is a flow chart showing a probe distribution mode switching method according to an embodiment.
FIG. 4 is a schematic block diagram illustrating a tumor therapy instrument according to an embodiment.

### DETAILED DESCRIPTION

In order to facilitate the understanding of the present application, the present application will be described in a more comprehensive manner with reference to the relevant accompanying drawings. Preferred embodiments of the present invention are shown in the accompanying drawings. However, the present application can be implemented in many different forms and is not limited to the embodiments described herein. Rather, the purpose of providing these embodiments is to make the disclosure of present application more thorough and comprehensive.

Unless otherwise defined, all technical and scientific terms used herein have the same meaning as commonly understood by those skilled in the technical field of the present application. The terms used in the description of the present application herein is only for the purpose of describing specific embodiments, and is not intended to limit the present application. The term "and/or" used herein includes any and all combinations of one or more related listed items.

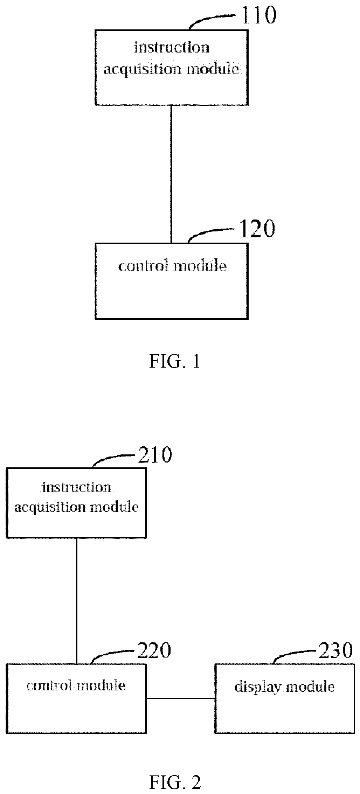

In an embodiment, as shown in FIG. 1, a probe distribution mode switching system is provided, which includes an instruction acquisition module 110 and a control module 120 connected to the instruction acquisition module 110.

The instruction acquisition module 110 receives a probe distribution mode instruction and transmits the probe distribution mode instruction to the control module 120; the control module 120 acquires a to-be-switched probe distribution mode corresponding to the probe distribution mode instruction, and switches a current probe distribution mode to the to-be-switched probe distribution mode.

The instruction acquisition module 110 refers to a device module with functions such as data receiving and sending; and the instruction acquisition module 110 can be an interface configured to transmit data. The control module 120 refers to a processing device with functions such as data processing and data transmission. For example, a processor included in the control module 120 can be an ARM processor. The probe distribution mode instruction can be configured to instruct the control module to start a corresponding probe distribution mode. The to-be-switched probe distribution mode refers to a probe distribution mode corresponding to the probe distribution mode instruction. The current probe distribution mode refers to a probe distribution mode currently performed by the probe distribution mode switching system. It should be noted that the probe distribution mode refers to a manner of performing probe distribution processing in an ablation region; and the probe distribution mode can be an executive program for the probe distribution processing.

Specifically, based on the connection of the control module 120 with the instruction acquisition module 110, the instruction acquisition module 110 can receive the probe distribution mode instruction transmitted by an external device, and transmit the received probe distribution mode instruction to the control module 120. The control module 120 can receive the probe distribution mode instruction, and acquire the to-be-switched probe distribution mode corresponding to the probe distribution mode instruction, and then can switch the current probe distribution mode to the to-be-switched probe distribution mode, so that the system performs the process of the probe distribution processing based on the to-be-switched probe distribution mode, thereby implementing the automatic switching of the probe distribution mode of the system.

Further, the user can operate the external device to make the external device generate the probe distribution mode instruction, and transmit the probe distribution mode instruction to the instruction acquisition module 110, and then the instruction acquisition module 110 can receive the probe distribution mode instruction and transmit the received probe distribution mode instruction to the control module 120. The control module 120 can process the received probe distribution mode instruction and acquire the to-be-switched probe distribution mode corresponding to the probe distribution mode instruction. The control module 10 can further query the probe distribution mode currently performed by the system to obtain the current probe distribution mode. If the current probe distribution mode is different from the to-be-switched probe distribution mode, the system performs the to-be-switched probe distribution mode after the current probe distribution mode is switched to the to-be-switched probe distribution mode. If the current probe distribution mode is the same as the to-be-switched probe distribution mode, the system can continue to perform the current probe distribution mode, and the system can also switch the current probe distribution mode to the to-be-switched probe distribution mode, and the system continues to perform the current probe distribution mode.

In the above-mentioned probe distribution mode switching system, the probe distribution mode instruction is received through the instruction acquisition module; the control module acquires the to-be-switched probe distribution mode corresponding to the probe distribution mode instruction according to the probe distribution mode instruction; and then the control module can switch the current probe distribution mode to the to-be-switched probe distribution mode, thereby implementing the switching of the probe distribution mode. By automatically switching the mode according to the probe distribution mode instruction, the probe distribution manner is diversified and the convenience of products is improved.

In an embodiment, the control module compares a priority of the to-be-switched probe distribution mode to a priority of the current probe distribution mode; when the priority of the to-be-switched probe distribution mode is greater than the priority of the current probe distribution mode, the current probe distribution mode is switched to the to-be-switched probe distribution mode.

Specifically, a priority can be preset for the probe distribution mode, and the probe distribution mode with a high priority can be prioritized. After acquiring the to-be-switched probe distribution mode and the current probe distribution mode, the control module can query the priority of the to-be-switched probe distribution mode and the priority of the current probe distribution mode, and compare the priority of the to-be-switched probe distribution mode to the priority of the current probe distribution mode. When the priority of the to-be-switched probe distribution mode is greater than the priority of the current probe distribution mode, the control module switches the current probe distribution mode to the to-be-switched probe distribution mode.

In a specific example, the control module keeps the system to perform the current probe distribution mode when the priority of to-be-switched probe distribution mode is less than the priority of the current probe distribution mode. When the priority of to-be-switched probe distribution mode is the same as the priority of the current probe distribution mode, the control module does not perform the switching, such that the system maintains the current probe distribution mode; alternatively, the current probe distribution mode is switched to the to-be-switched probe distribution mode, while the system still maintains the current probe distribution mode.

In the above-mentioned probe distribution mode switching system, by comparing priorities of probe distribution modes provided with the priorities, the switching of the probe distribution mode is performed when a priority condition is met, thereby improving the flexibility of the switching of the probe distribution mode and improving the convenience of the product.

In an embodiment, the to-be-switched probe distribution mode is an automatic probe distribution mode or a manual probe distribution mode.

The automatic probe distribution mode refers to that the automatic probe distribution processing of the system can be implemented by executing a corresponding program. The manual probe distribution process refers to that a user is required to manually adjust the operation process during the probe distribution process of the system (for example, to manually adjust the position of the probe, the voltage of the probe and the selection and preservation of the ablation region, etc.).

Specifically, when the to-be-switched probe distribution mode is the automatic probe distribution mode, and the control module switches the current probe distribution mode to the automatic probe distribution mode, the working process of the system performing the automatic probe distribution mode is as follows: according to an operating parameter inputted by the user, the database is queried to obtain the corresponding simulated tumor pattern and the pattern type corresponding to the simulated tumor pattern; based on the pattern type, the database is queried and the number of probes and the positions of the probes corresponding to the pattern type are obtained from the database; and different pattern type corresponds to different number of probes and different position arrangement of probes; and then the number of probes and the position arrangement of the probes can be determined according to the type of tumor. By selecting simulated probe groups with the number of probes, and sequentially arranging each simulated probe group in the corresponding position in the simulated tumor pattern according to the positions of the probes, and modeling each simulated probe group according to a corresponding application scenario, the probe group is equivalent to a two-dimensional plane point, the ablation region is equivalent to a plane with a corresponding area, and a voltage is applied between the probes to obtain a Laplace equation and a boundary condition thereof, and the equation is solved to obtain a distribution formula of an electric field intensity; and then a contour graph of the electric field intensity is drawn through a corresponding contour graph algorithm. The contour graph is the ablation region; and then a simulated ablation processing is performed on the profile graphic of the tumor according to the ablation region, thereby implementing the process of the automatic probe distribution.

When the to-be-switched probe distribution mode is the manual probe distribution mode, and the control module switches the current probe distribution mode to the manual probe distribution mode, the working process of the system performing the manual probe distribution mode is as follows: the user can input the operating parameter to make the system generate and display the corresponding simulated tumor pattern and the ablation region; the user can observe the simulated tumor pattern and ablation region, and manually add or delete the probe, and move the added probe to make the ablation region formed by the combination of each probe satisfy the ablation condition, and then the simulated ablation processing can be performed on the profile graphic of the tumor according to the ablation region, thereby implementing the process of the manual probe distribution process.

Further, the working process of the system performing the manual probe distribution mode can also be as follows: if the simulated tumor pattern is larger and only two probes are used, the user can save the ablation region, then move the probe to form a new ablation region, and then save and move again, such operation is repeated in this way to obtain an ablation region that satisfies the ablation condition. The simulated ablation processing is performed on the profile graphic of the tumor through the ablation region to implement the process of the manual probe distribution.

In an embodiment, the control module parses the probe distribution mode instruction, and performs switching of a corresponding mode according to the parsing result.

Specifically, the probe distribution mode instruction can be an automatic probe distribution mode instruction corresponding to the automatic probe distribution mode, or a manual probe distribution mode instruction corresponding to the manual probe distribution mode. After receiving the probe distribution mode instruction, the control module can parse the received probe distribution mode instruction to obtain the parsing result. According to the parsing result, the control module can acquire the automatic probe distribution mode corresponding to the automatic probe distribution mode instruction when the probe distribution mode instruction is the automatic probe distribution mode instruction, and switch the current probe distribution mode to the automatic probe distribution mode; and according to the parsing result, the control module can acquire the manual probe distribution mode corresponding to the manual probe distribution mode instruction when the probe distribution mode instruction is the manual probe distribution mode instruction, and switch the current probe distribution mode to the manual probe distribution mode. Accordingly, the automatic switching of the probe distribution mode is implemented, and probe distribution manners are diversified.

In an embodiment, the control module is configured to, when acquiring an initialization instruction, start the automatic probe distribution mode.

The initialization instruction can be configured to instruct the system to initialize a probe distribution. The initialization instruction can be obtained through operating an external device by a user.

Specifically, the instruction acquisition module can receive the initialization instruction obtained by the operation of the user, and transmit the initialization instruction to the control module; the control module can, when acquiring the initialization instruction, start the automatic probe distribution mode, so that the system can automatically perform the probe distribution mode when entering the probe distribution interface, thereby implementing the process of the automatic probe distribution.

In an embodiment, as shown in FIG. 2, a probe distribution mode switching system is provided, which includes an instruction acquisition module 210 and a control module 220 connected to the instruction acquisition module 210, and further includes a display module 230.

The display module 230 displays an automatic probe distribution interface corresponding to the automatic probe distribution mode, and displays a manual probe distribution interface corresponding to the manual probe distribution mode.

The display module 230 can be a displayer, for example, the display module 230 can be a liquid crystal displayer or an LED displayer.

Specifically, based on the connection of the display module 230 with the control module 220, the control module 220 can transmit data obtained by processing (such as relevant data corresponding to the automatic probe distribution mode and relevant data corresponding to the manual probe distribution mode) to the display module 230, and then the automatic probe distribution interface corresponding to the automatic probe distribution mode and the manual probe distribution interface corresponding to the manual probe distribution mode can be displayed through the display module 230, thereby improving the convenience of the system.

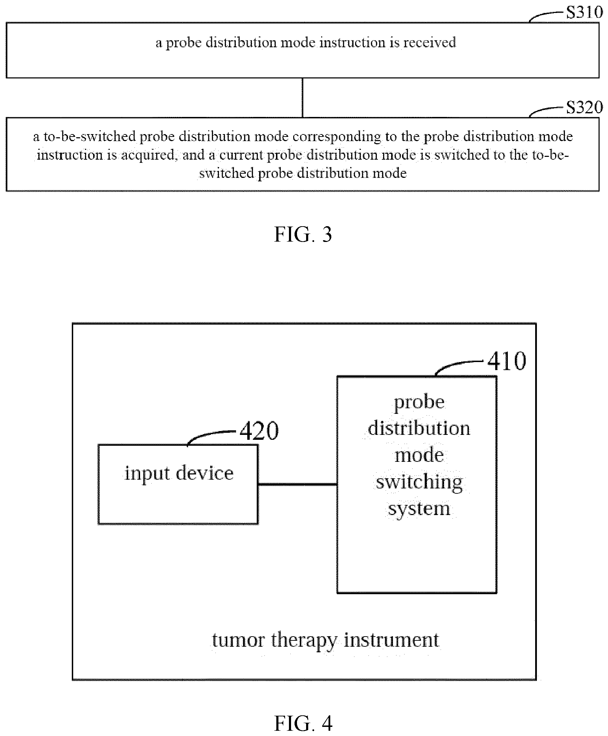

In an embodiment, as shown in FIG. 3, a probe distribution mode switching method is provided, which includes the following steps.

Step S310: a probe distribution mode instruction is received.

Step S320: a to-be-switched probe distribution mode corresponding to the probe distribution mode instruction is acquired, and a current probe distribution mode is switched to the to-be-switched probe distribution mode.

Specifically, the instruction acquisition module receives the probe distribution mode instruction; the control module acquires the to-be-switched probe distribution mode corresponding to the probe distribution mode instruction according to the probe distribution mode instruction; and then the control module can switch the current probe distribution mode to the to-be-switched probe distribution mode, to implement the switching of the probe distribution mode. By automatically switching the mode according to the probe distribution mode instruction, the probe distribution manner is diversified and the convenience of the product is improved.

It should be understood that although the various steps in the flow chart of FIG. 3 are displayed in sequence as indicated by the arrows, these steps are not definitely performed in sequence in the order indicated by the arrows. Unless specifically stated in this article, the execution of these steps is not strictly limited in order, and these steps can be executed in other orders. Moreover, at least part of the steps in FIG. 3 can include multiple sub-steps or multiple stages. These sub-steps or stages are not definitely executed at the same time, but can be executed at different time. The execution of these sub-steps or stages is not definitely executed in sequence, but may be performed in turns or alternately with other steps or at least a part of sub-steps or stages of other steps.

In an embodiment, as shown in FIG. 4, a tumor therapy instrument is provided, which includes the probe distribution mode switching system 410 of any one of the above embodiments.

The tumor therapy instrument may be a tumor therapy instrument with a high-voltage electric pulse.

Specifically, the tumor therapy instrument can include a probe distribution mode switching system 410. After the probe distribution mode switching system 410 starts working, the probe distribution mode instruction can be received through the instruction acquisition module included in the probe distribution mode switching system 410; the to-be-switched probe distribution mode corresponding to the probe distribution mode instruction can be acquired by the control module included in the probe distribution mode switching system 410; and the control module can switch the current probe distribution mode to the to-be-switched probe distribution mode, to implement the switching of the probe distribution mode. Furthermore, the tumor therapy instrument can perform the switched probe distribution mode to implement the process of the probe distribution.

In a specific embodiment, as shown in FIG. 4, an input device 420 for inputting a probe distribution mode instruction is further included.

The input device 420 may be, but is not limited to, a button and a touch screen.

Specifically, based on the connection of the input device 420 to the probe distribution mode switching system 410, the user can operate the input device 420 so that the input device 420 can transmit the probe distribution mode instruction to the probe distribution mode switching system 410, and then the probe distribution mode switching system 410 can receive the probe distribution mode instruction through the instruction acquisition module; the to-be-switched probe distribution mode corresponding to the probe distribution mode instruction can be acquired through the control module, and then the control module can switch the current probe distribution mode to the to-be-switched probe distribution mode, thereby implementing the switching of the probe distribution mode and improving the convenience of the product.

Those of ordinary skill in the art can understand that all or part of the process in the above-mentioned embodiment method can be implemented by instructing relevant hardware through a computer program. The computer program can be stored in a non-transitory computer readable storage. The computer program, when executed, may include the processes of the above method embodiments. Any reference to memory, storage, database or other media used in the embodiments provided in this application may include non-transitory and/or transitory memory. The non-transitory memory may include a read only memory (ROM), a programmable ROM (PROM), an electrically programmable ROM (EPROM), an electrically erasable programmable ROM (EEPROM), or a flash memory. The transitory memory may include the random access memory (RAM) or an external cache memory. As an illustration and not a limitation, RAM is available in many forms, such as static RAM (SRAM), dynamic RAM (DRAM), synchronous DRAM (SDRAM), double data rate SDRAM (DDRSDRAM), enhanced SDRAM (ESDRAM), synchronous link (Synchlink) DRAM (SLDRAM), Rambus direct RAM (RDRAM), direct rambus dynamic RAM (DRDRAM), and rambus dynamic RAM (RDRAM), etc.

The technical features of the above-mentioned embodiments can be combined arbitrarily. In order to make the description concise, all possible combinations of the various technical features in the above-mentioned embodiments are not described. However, as long as there is no contradiction in the combination of these technical features, all should be considered as the scope of the description.

The above-mentioned embodiments are merely some embodiments of the present application, and the description is relatively specific and detailed, but it should not be understood as a limitation on the scope of the present application. It should be pointed out that those of ordinary skill in the art can make several modifications and improvements without departing from the concept of the present application, and these all fall within the protection scope of this application. Therefore, the scope of protection of the present invention shall be subject to the appended claims.

## Claims

1. A probe distribution mode switching system, **characterized by** comprising an instruction acquisition module and a control module connected to the instruction acquisition module; wherein
the instruction acquisition module is configured to receive a probe distribution mode instruction, and transmit the probe distribution mode instruction to the control module;
the control module is configured to acquire a to-be-switched probe distribution mode corresponding to the probe distribution mode instruction, and switch a current probe distribution mode to the to-be-switched probe distribution mode.

2. The probe distribution mode switching system according to claim 1, wherein,
the control module is configured to compare a priority of the to-be-switched probe distribution mode to a priority of the current probe distribution mode, and switch the current probe distribution mode to the to-be-switched probe distribution mode when the priority of the to-be-switched probe distribution mode is greater than the priority of the current probe distribution mode.

3. The probe distribution mode switching system according to claim 2, wherein,
the control module is configured to perform the current probe distribution mode when the priority of to-be-switched probe distribution mode is less than the priority of the current probe distribution mode.

4. The probe distribution mode switching system according to claims 1 to 3, wherein,
the to-be-switched probe distribution mode is an automatic probe distribution mode or a manual probe distribution mode.

5. The probe distribution mode switching system according to claim 4, wherein,
the control module is configured to parse the probe distribution mode instruction, and perform switching of a corresponding mode according to a parsing result.

6. The probe distribution mode switching system according to claim 4, wherein,
the control module is configured to, when acquiring an initialization instruction, start the automatic probe distribution mode.

7. The probe distribution mode switching system according to claim 4, further comprising a display module, wherein
the display module is configured to display an automatic probe distribution interface corresponding to the automatic probe distribution mode, and display a manual probe distribution interface corresponding to the manual probe distribution mode.

8. A probe distribution mode switching method, **characterized by** comprising:
receiving a probe distribution mode instruction;
acquiring a to-be-switched probe distribution mode corresponding to the probe distribution mode instruction, and switching a current probe distribution mode to the to-be-switched probe distribution mode.

9. A tumor therapy instrument, comprising the probe distribution mode switching system according to any one of claims 1 to 7.

10. The tumor therapy instrument according to claim 9, further comprising an input device configured to input the probe distribution mode instruction; wherein
the input device is connected to the probe distribution mode switching system.
